# EUROPEAN PATENT APPLICATION

(11) **EP 4 101 491 A1**
(43) Date of publication of application: **14.12.2022**
(21) Application number: 21750884.5
(22) Date of filing: 29.01.2021
(51) Int. Cl.: A61M 25/00

(54) **MEDICAL TUBE AND CATHETER**

(30) Priority: 04.02.2020 JP 2020016945
(71) Applicant: Asahi Intecc Co., Ltd., Seto-shi, Aichi 489-0071 (JP)
(72) Inventor: IHARA, Kosei, Seto-shi, Aichi 489-0071 (JP)
(74) Representative: Winter, Brandl - Partnerschaft mbB
(86) International application number: PCT/JP2021/003418
(87) International publication number: WO 2021/157499

(57) **Abstract**

To reinforce a layer that is configured to contain fluororesin while suppressing the thickness of the entire medical tube. A medical tube 11 includes a first resin layer 20 that is configured to contain fluororesin, and a second resin layer 30 that is configured to coat an outer peripheral surface 22 of the first resin layer 20 and containing polyimide resin. The thickness T2 of the second resin layer 30 is larger than 0 µm but not exceeding 15 µm.

## Description

### TECHNICAL FIELD

The disclosed embodiments relate to a medical tube and a catheter.

### BACKGROUND

Patent Literature 1 discloses a catheter provided with a sheath. The sheath is a long member, which includes an inner layer having therein a main lumen and an outer layer coating the inner layer. Patent Literature 1 describes that a fluorine thermoplastic polymer material is used for the inner layer, and that the thickness of the outer layer is approximately 50 µm to 150 µm.

### CITATION LIST

### Patent Literature

Patent Literature 1: Japanese Patent Application Laid-Open No. 2016-214942

### SUMMARY

### Technical Problem

A layer that is configured to contain fluororesin has a problem of low tensile strength. In addition, in the configuration disclosed in Patent Literature 1, the thickness of the entire sheath may be large because the thickness of the outer layer is approximately 50 µm to 150 µm.

The disclosed embodiments aim at providing a technology of reinforcing a layer that is configured to contain fluororesin while suppressing the thickness of the entire medical tube.

### Solution to Problem

The medical tube of the disclosed embodiments includes a first resin layer that is configured to contain fluororesin, and a second resin layer coating the outer peripheral surface of the first resin layer and that is configured to contain polyimide resin. The thickness of the second resin layer is larger than 0 µm but not exceeding 15 µm.

In the disclosed embodiments, with the second resin layer that is configured to contain polyimide resin, it is possible to reinforce the first resin layer that is configured to contain fluororesin by the second resin layer. Moreover, as the thickness of the second resin layer does not exceed 15 µm, the thickness of the medical tube can be reduced.

### BRIEF DISCRIPTION OF DRAWINGS

FIG. 1 is an overall view of a catheter according to a first embodiment.
FIG. 2 is a sectional view cut along the longitudinal direction of a hollow shaft of FIG. 1.
FIG. 3 is a sectional view along an A-A section of FIG. 2.
FIG. 4 is a sectional view cut along the longitudinal direction of a hollow shaft according to a second embodiment.

### DESCRIPTION OF EMBODIMENTS

Preferred embodiments of the present disclosure will be described.

A third resin layer coating the outer peripheral surface of the second resin layer may be further included, and the thickness of the third resin layer may be larger than the thickness of the second resin layer. With this configuration, it is possible to exert the characteristics of the resin forming the third resin layer and particularly impart the pressure resistance and flexibility.

The third resin layer may include a reinforcing layer embedded therein. With this configuration, it is possible to impart the pressure resistance, kink resistance, and torquability.

The distal end portion may be more flexible than another part positioned on the more proximal end side than the distal end portion. With this configuration, it is possible to secure the followability and safety of a combined device at the distal end portion.

The catheter of the disclosed embodiments may include the above-described medical tube. With this configuration, it is possible to provide the catheter with a small thickness in which the first resin layer that is configured to contain fluororesin is reinforced by the second resin layer.

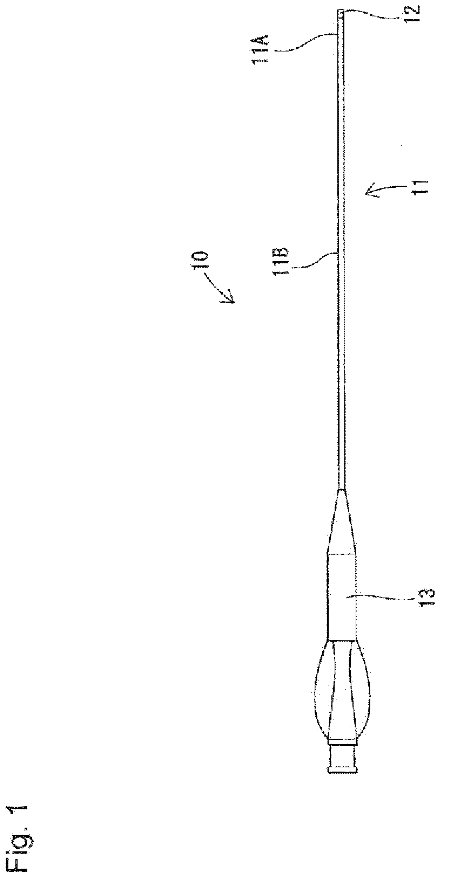

Next, the first embodiment embodying the present disclosure will be described with reference to FIG. 1 to FIG. 3. FIG. 1 illustrates a catheter 10 using the medical tube of the embodiment. In FIG 1, the right side is the distal end side (far side) inserted into a body, and the left side is the rear end side (near side, proximal end side) operated by a technician such as a doctor.

The catheter 10 includes a hollow shaft 11, a distal tip 12 joined to the distal end side, and a connector 13 joined to the proximal end side of the hollow shaft 11, as illustrated in FIG. 1. The hollow shaft 11 is tubular, and has a lumen 15. A medical device such as a guide wire is inserted to the lumen 15. A distal end portion 11A of the hollow shaft 11 is more flexible than the other part 11B positioned on the more proximal end side than the distal end portion 11A. The hollow shaft 11 is an example of the medical tube.

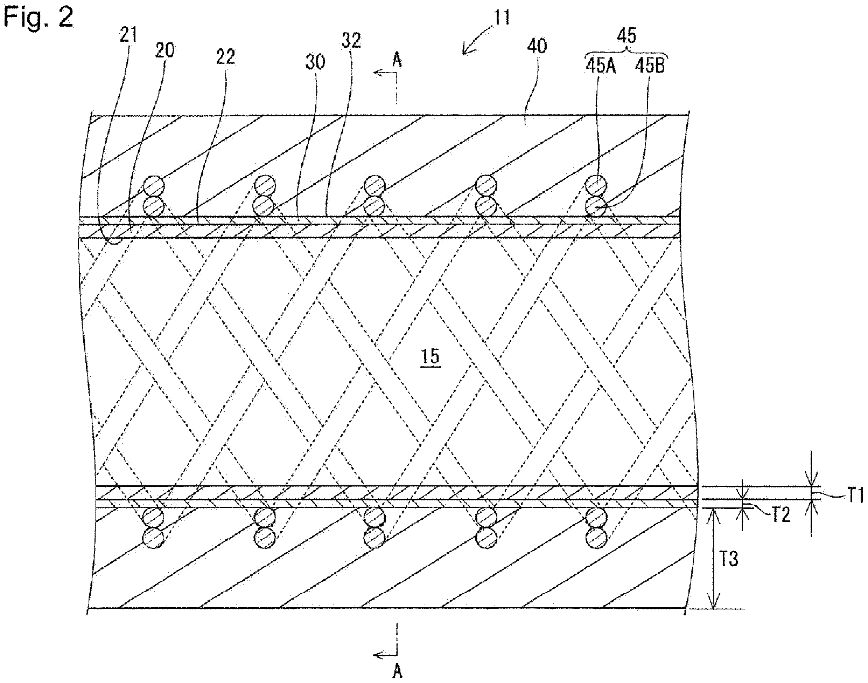

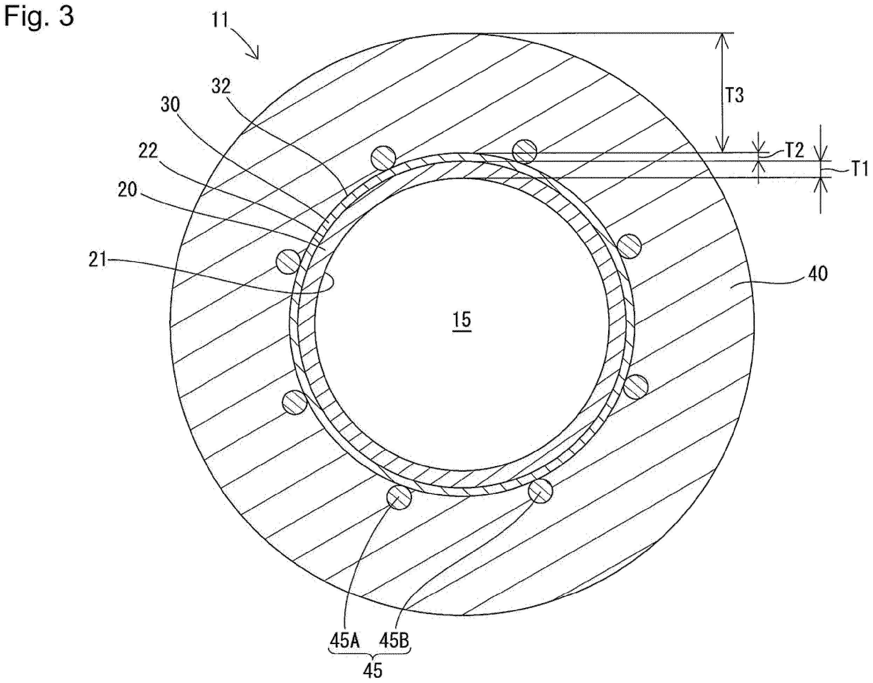

The hollow shaft 11 includes a first resin layer 20, a second resin layer 30, and a third resin layer 40, as illustrated in FIG. 2 and FIG. 3. The first resin layer 20 forms an innermost layer of the hollow shaft 11, and includes an inner peripheral surface 21 facing the lumen 15. The second resin layer 30 coats an outer peripheral surface 22 of the first resin layer 20. That is, the first resin layer 20 and the second resin layer 30 are directly in contact. The third resin layer 40 coats an outer peripheral surface 32 of the second resin layer 30. The hollow shaft 11 has a multilayer structure in which the first resin layer 20, the second resin layer 30, the third resin layer 40 are layered in this order from the inner side.

The first resin layer 20 is configured to contain fluororesin. The first resin layer 20 is preferably formed of a resin material excellent in slidability and chemical resistance. The fluororesin is a general term of synthetic resin containing fluorine, and is thermoplastic resin containing fluorine, fluoroelastomer, or the like. The examples of such a resin material include PTFE (polytetrafluoroethylene), PFA (tetrafluoroethylene/perfluoroalkylvinyl ether copolymer), FEP (tetrafluoroethylene/hexafluoropropylene copolymer), and the like. Note that the first resin layer 20 may contain arbitrary components other than fluororesin but preferably contains 90 mass% or more fluororesin.

The thickness T1 of the first resin layer 20 is not particularly limited. However, the thickness T1 is preferably not smaller than 5 µm but not exceeding 30 µm, more preferably not smaller than 6 µm but not exceeding 20 µm, and still more preferably not smaller than 7 µm but not exceeding 15 µm. If the thickness T1 of the first resin layer 20 is not smaller than the lower limit value of the above-described range, the slidability and chemical resistance of the inner surface can be secured, which is preferable. If the thickness T1 of the first resin layer 20 does not exceed the upper limit value of the above-described range, it is possible to reduce the thickness of the hollow shaft 11, secure the inner diameter of the lumen 15, and reduce the outer diameter of the hollow shaft 11.

The second resin layer 30 is configured to contain polyimide resin. The second resin layer 30 is preferably formed of a resin material having higher rigidity than the first resin layer 20. The second resin layer 30 is more preferably formed of a resin material more excellent in pressure resistance and voltage resistance than the first resin layer 20. The polyimide resin is a polymer having an imide bond as the main chain. The concrete examples include polyimide, polyamidimide, polyesterimide, polyetherimide, and the like. Such resin are usually used individually. However, two or more kinds of resin may be mixed for use. Among polyimide resin, polyimide, which is excellent particularly in dynamic characteristics, is preferably used. Among polyimide, aromatic polyimide is more preferably used. This aromatic polyimide may be thermoplastic or non-thermoplastic. Note that the second resin layer 30 may include arbitrary components other than polyimide resin but preferably contains 90 mass% or more polyimide resin.

The hardness of the material for the second resin layer 30 is preferably not lower than 72D but not exceeding 100D, as the shore hardness, more preferably not lower than 75D but not exceeding 95D, and still more preferably not lower than 78D but not exceeding 90D. The numerical value of such shore hardness may be calculated by converting another hardness such as Rockwell hardness into shore hardness. If the shore hardness is not lower than the lower limit value, it is possible to sufficiently reinforce the first resin layer 20. If the shore hardness does not exceed the upper limit value, it is advantageous in the aspect of material costs.

The thickness T2 of the second resin layer 30 is larger than 0 µm but not exceeding 15 µm. The thickness T2 of the second resin layer 30 is more preferably not smaller than 1 µm but not exceeding 11 µm, and preferably not smaller than 1 µm but not exceeding 10 µm, still more preferably not smaller than 2 µm but not exceeding 6 µm, and most preferably not smaller than 2 µm but not exceeding 5 µm. It is preferable in the viewpoint of reinforcing the first resin layer 20 that the thickness T2 of the second resin layer 30 is larger than 0 and not smaller than the lower limit value of the above-described range. If the thickness T2 of the second resin layer 30 does not exceed the upper limit value of the above-described range, it is possible to reduce the thickness of the hollow shaft 11, secure the inner diameter of the lumen 15, and reduce the outer diameter of the hollow shaft 11. Moreover, if the thickness T2 of the second resin layer 30 does not exceed the upper limit value of the above-described range, the processing of folding a tube and cutting a tube, for example, are performed easily.

The thickness T2 of the second resin layer 30 is substantially constant in the longitudinal direction and the circumferential direction of the second resin layer 30. In other words, the second resin layer 30 does not include any structure embedded therein, and does not have any portion partially cut out or thinned in the circumferential direction. With such a configuration, it is possible to stably exert the action of reinforcing the first resin layer 20 by the second resin layer 30 and the action of improving the voltage resistance of the hollow shaft 11 in the longitudinal direction and the circumferential direction.

The examples of the resin forming the third resin layer 40 include polyamide resin, polyester resin, polyurethane resin, polyolefin resin, aromatic polyether ketone resin, polycarbonate resin, and the like. Only one of these kinds may be used, or two or more kinds may be used together. The polyamide resin is exemplified by polyamide and polyamide elastomer. The polyurethane resin is exemplified by polyurethane and polyurethane elastomer. The polyester resin is exemplified by polybutylene terephthalate and polyester elastomer. The polyolefin resin is exemplified by polyethylene, polypropylene, and ethylene-propylene copolymer. The aromatic polyether ketone resin is exemplified by polyether ether ketone (PEEK). The third resin layer 40 is preferably configured to include resin more flexible than the second resin layer 30. The third resin layer 40 is preferably configured to include polyamide resin from the viewpoint of moldability and flexibility. Note that the third resin layer 40 may include arbitrary components other than polyamide resin but preferably contains 90 mass% or more polyamide resin.

The thickness T3 of the third resin layer 40 is larger than the thickness T2 of the second resin layer 30. The thickness T3 of the third resin layer 40 is preferably not smaller than 20 µm but not exceeding 200 µm, more preferably not smaller than 25 µm but not exceeding 150 µm, and still more preferably not smaller than 30 µm but not exceeding 100 µm. Note that the thickness T3 of the third resin layer 40 is measured at a region where a reinforcing layer 45 described later is not provided. If the thickness T3 of the third resin layer 40 is not smaller than the lower limit value of the above-described range, the rigidity of the hollow shaft 11 can be secured. If the thickness T3 of the third resin layer 40 does not exceed the upper limit value of the above-described range, it is possible to reduce the thickness of the hollow shaft 11, secure the inner diameter of the lumen 15, and reduce the outer diameter of the hollow shaft 11.

The hardness of the material for the third resin layer 40 is preferably not lower than 25D but not exceeding 80D, as the shore hardness, more preferably not lower than 30D but not exceeding 75D, and still more preferably not lower than 35D but not exceeding 69D. The numerical value of such shore hardness may be calculated by converting another hardness such as Rockwell hardness into shore hardness. If the shore hardness is not lower than the lower limit value, the rigidity of the hollow shaft 11 can be secured. If the shore hardness does not exceed the upper limit value, the flexibility of the hollow shaft 11 can be secured.

The third resin layer 40 includes the reinforcing layer 45 embedded therein. The reinforcing layer 45 is a braid formed by mutually braiding a plurality of wires 45A, 45B. The wire 45A is wound in the right direction toward the distal end side. The wire 45B is wound in the left direction toward the distal end side. The material for the wires 45A, 45B is not particularly limited. The wires 45A, 45B may be formed of stainless steel (SUS304), tungsten, or superelastic alloy such as a Ni-Ti alloy, or may be formed of resin having higher hardness than the third resin layer 40, such as reinforced plastic. The reinforcing layer 45 is arranged at a position adjacent to or close to the outer peripheral surface 32 of the second resin layer 30 in the third resin layer 40.

Next, a method of producing the above-described hollow shaft 11 will be described. The hollow shaft 11 includes a step of forming the first resin layer 20 around a core wire, a step of immersing the first resin layer 20 in liquid containing the material for the second resin layer 30, a step of pulling out the first resin layer 20 from the liquid containing the material for the second resin layer 30, a step of firing the material for the second resin layer 30 attached around the first resin layer 20 for imidization, and a step of removing the core wire from the first resin layer 20. As the liquid containing the material for the second resin layer 30, there can be used a solution formed by dissolving polyamic acid in a solvent or a solution formed by dissolving a polyimide raw material in a solvent. The thickness of the second resin layer 30 can be adjusted by changing the concentration, surface tension, viscosity of the liquid containing the material for the second resin layer 30, and a speed of pulling out the first resin layer 20 from the liquid containing the material for the second resin layer 30, and the like.

There is known a method of producing a tube by performing stretching treatment on a tube formed of PTFE to increase the tensile strength. However, the tensile strength increased by stretching treatment is limited. Moreover, in the case of producing a tube formed of PTFE by stretching treatment, the tube after stretching treatment may have a void defect or unevenness in thickness. Such a void defect or unevenness in thickness may cause deterioration of tensile strength and pressure resistance of the tube formed of PTFE. Meanwhile, in the method of producing the hollow shaft 11 of the embodiment, the first resin layer 20 is reinforced by coating the outer peripheral surface 22 of the first resin layer 20 with the second resin layer 30, without performing stretching treatment on the first resin layer 20. In this configuration, it is possible to achieve sufficient tensile strength of the tube including the first resin layer 20 and the second resin layer 30, and eliminate the possibility of causing problems of a void defect or unevenness in thickness in the first resin layer 20.

Next, the effects of the embodiment will be described. In this embodiment, with the second resin layer 30 that is configured to contain polyimide resin, it is possible to reinforce the first resin layer 20 that is configured to contain fluororesin by the second resin layer 30. Moreover, the thickness of the second resin layer 30 is not larger than 15 µm, which suppresses the thickness of the hollow shaft 11. Furthermore, in the embodiment, the first resin layer 20 is configured to contain fluororesin, which improves the slidability and the chemical resistance of the inner peripheral surface 21. Moreover, the second resin layer 30 is configured to contain imide resin, which improves the pressure resistance and the voltage resistance of the hollow shaft 11.

The embodiment further includes the third resin layer 40 coating the outer peripheral surface 32 of the second resin layer 30 and that is configured to contain resin more flexible than the second resin layer 30. The thickness of the third resin layer 40 is larger than the thickness of the second resin layer 30. Therefore, it is possible to exert the characteristics of the resin forming the above-described third resin layer 40 and particularly impart the pressure resistance and flexibility.

In the embodiment, the third resin layer 40 includes the reinforcing layer 45 embedded therein. Therefore, it is possible to impart the pressure resistance, kink resistance, and torquability.

In this embodiment, the distal end portion 11A is more flexible than the other part 11B positioned on the more proximal end side than the distal end portion 11A. Therefore, it is possible to secure the followability and safety of a combined device at the distal end portion 11A.

The catheter 10 of the embodiment includes the above-described hollow shaft 11. Therefore, it is possible to provide the catheter 10 with a small thickness in which the first resin layer 20 that is configured to contain fluororesin is reinforced by the second resin layer 30.

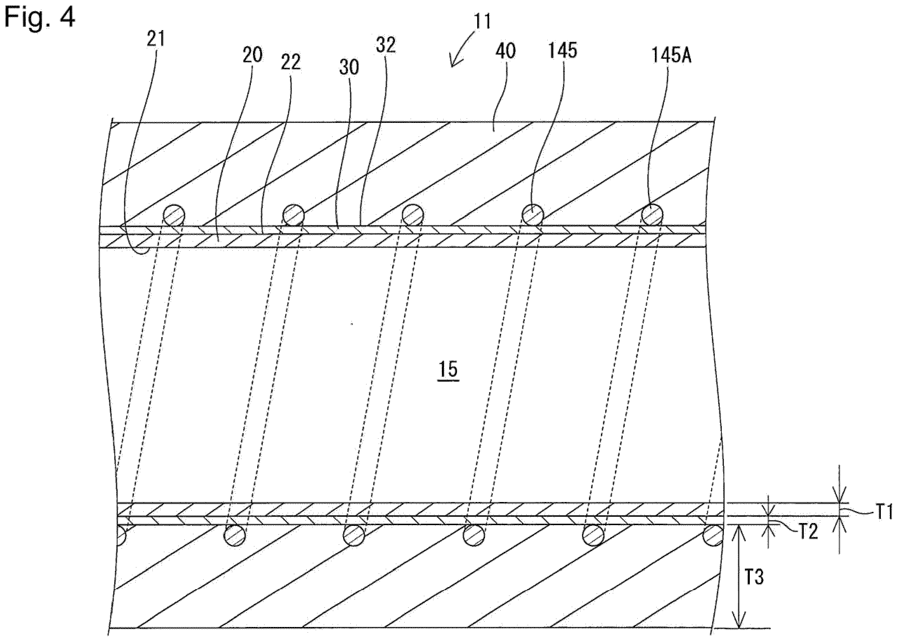

Next, another embodiment embodying the present disclosure will be described. A reinforcing layer 145 of the second embodiment is a coil body formed by winding a wire 145A, as illustrated in FIG. 4. The wire 145A is wound in the right direction toward the distal end side. The material for the wire 145A is not particularly limited, and may be the same as that for the reinforcing layer 45. The reinforcing layer 145 is arranged at a position adjacent to or close to the outer peripheral surface 32 of the second resin layer 30 in the third resin layer 40.

Moreover, in the longitudinal direction of the hollow shaft, a position and a range where the second resin layer is provided can be appropriately designed. For example, the hollow shaft may be configured to have the second resin layer in the entire range in the longitudinal direction, or may be configured to have the second resin layer at another part without having the second resin layer at the distal end portion.

Other than the above-described embodiments, the configuration of the hollow shaft can be appropriately changed. For example, the hollow shaft may not have the third resin layer, and the outer peripheral surface of the second resin layer may be coated with a resin layer different from the third resin layer. In the hollow shaft, the reinforcing layer may not be provided at the distal end portion.

Other than the above-described embodiments, the kind of a catheter to which the medical tube is applied can be changed. For example, the catheter may be a balloon catheter.

The medical tube may be applied to a medical device other than a catheter. Such a medical device is exemplified by a tube for an indwelling needle.

### EXAMPLES

In the following, the description will be given more concretely using examples.

### 1. Formation of samples

There were formed samples 1 to 10 of a tube including the first resin layer and the second resin layer, and samples 11 to 15 of a single layer tube including the first resin layer. The samples 1 to 10 are examples, and the samples 11 to 15 are comparative examples. The first resin layer was formed of PTFE. The second resin layer was formed of polyimide. The thicknesses (µm) of the first resin layer and the second resin layer are as shown in Table 1. In the samples 1 to 10, the thickness of the second resin layer was larger than 0 µm but not exceeding 15 µm. To be more specific, the thickness of the second resin layer was larger than 0 µm but was smaller than 10 µm in the samples 1 to 5, while the thickness of the second resin layer was not smaller than 10 µm but not exceeding 15 µm in the samples 6 to 10.

### 2. Tensile characteristics test

For the samples 1 to 15, the tensile modulus, tensile stress, and tensile strain were measured by a method conforming to JIS K 7161-2014. Table 1 shows the results of the tensile modulus (MPa), the maximum value of tensile stress (tensile strength, MPa), and the maximum value of tensile strain (maximum strain, %). It can be evaluated that the strength of the tube is higher as the tensile modulus and the tensile strength are larger and the maximum strain is smaller.

### 3. Voltage resistance test

For the samples 1 to 15, the withstand voltage (kV) was detected using a spark tester (SHIN TOYO KIKI, K.K., Model No. HST-53KN1). A voltage applied on each sample is gradually increased, and the withstand voltage was evaluated as a voltage at the time when the spark tester reacted (a voltage of dielectric breakdown). Table 1 shows the results of withstand voltages. It can be evaluated that the voltage resistance of the tube is higher as the withstand voltage is higher.

### 4. Results

**[Table 1]**

| Sample No. | Thickness of first resin layer (µm) | Thickness of second resin layer (µm) | Tensile modulus (MPa) | Tensile strength (MPa) | Maximum strain (%) | Withstand voltage (kV) |
|---|---|---|---|---|---|---|
| 1 | 10 | 6 | 1253.00 | 86.08 | 38.24 | 0.44 |
| 2 | 10 | 5 | 1968.10 | 83.12 | 17.45 | 0.32 |
| 3 | 10 | 5 | 2267.50 | 87.47 | 54.27 | 0.43 |
| 4 | 10 | 6 | 2397.60 | 89.75 | 34.17 | 0.33 |
| 5 | 10 | 6 | 1988.20 | 74.43 | 66.33 | 0.84 |
| 6 | 10 | 11 | 1419.00 | 93.14 | 13.65 | 0.32 |
| 7 | 10 | 10 | 1958.00 | 89.33 | 13.14 | 0.28 |
| 8 | 10 | 10.5 | 1945.00 | 90.21 | 11.31 | 0.33 |
| 9 | 10 | 10.5 | 1783.00 | 91.25 | 12.73 | 0.26 |
| 10 | 10 | 10.5 | 1896.00 | 89.83 | 10.59 | 0.31 |
| 11 | 10 | 0 | 263.70 | 33.33 | 598.38 | 0.32 |
| 12 | 10 | 0 | 243.77 | 30.51 | 469.98 | 0.28 |
| 13 | 10 | 0 | 208.10 | 28.20 | 485.43 | 0.33 |
| 14 | 10 | 0 | 205.68 | 22.18 | 334.63 | 0.26 |
| 15 | 10 | 0 | 170.47 | 22.91 | 380.00 | 0.31 |

In the samples 1 to 5, the maximum value of the tensile modulus was 2397.60 MPa, and the average value thereof was 1974.88 MPa. In the samples 6 to 10, the maximum value of the tensile modulus was 1958.00 MPa, and the average value thereof was 1800.20 MPa. In the samples 11 to 15, the maximum value of the tensile modulus was 263.70 MPa, and the average value thereof was 218.34 MPa. The tensile moduluses of the samples 1 to 10 were higher than the tensile moduluses of the samples 11 to 15, which indicated that the second resin layer reinforced the first resin layer.

In the samples 1 to 5, the maximum value of the tensile strength was 89.75 MPa, and the average value thereof was 84.17 MPa. In the samples 6 to 10, the maximum value of the tensile strength was 93.14 MPa, and the average value thereof was 90.75 MPa. In the samples 11 to 15, the maximum value of the tensile strength was 33.33 MPa, and the average value thereof was 27.43 MPa. The tensile strengths of the samples 1 to 10 were higher than the tensile strengths of the samples 11 to 15, which indicated that the second resin layer reinforced the first resin layer.

In the samples 1 to 5, the maximum value of the maximum strain was 66.33%, and the average value thereof was 42.09%. In the samples 6 to 10, the maximum value of the maximum strain was 13.65%, and the average value thereof was 12.97%. In the samples 11 to 15, the maximum value of the maximum strain was 598.38%, and the average value thereof was 453.68%. The maximum strains of the samples 1 to 10 were smaller than the maximum strains of the samples 11 to 15, which indicated that the second resin layer reinforced the first resin layer.

In the samples 1 to 5, the maximum value of the withstand voltage was 0.84 kV%, and the average value thereof was 0.47 kV. In the samples 6 to 10, the maximum value of the withstand voltage was 1.15 kV%, and the average value thereof was 1.08 kV. In the samples 11 to 15, the maximum value of the withstand voltage was 0.33 kV, and the average value thereof was 0.30 kV. The withstand voltage for the samples 1 to 10 was higher than the withstand voltage for the samples 6 to 10, which indicated that the second resin layer improved the voltage resistance of the medical tube.

### (Samples 16 and 17)

There were formed a sample 16 of a tube including the first resin layer (PTFE) and the second resin layer (polyimide), and a sample 17 of a single layer tube including the first resin layer (PTFE). The sample 16 is an example, and the sample 17 is a comparative example. In the samples 16 and 17, the thickness of the first resin layer was 10 µm, and the outer diameter was 0.62 mm. In the sample 16, the thickness of the second resin layer was approximately 3 µm. In the sample 16, the tensile modulus was 1053.27 MPa, and the tensile strength was 48.30 MPa. Meanwhile, in the sample 17, the tensile modulus was 94.59 MPa, and the tensile strength was 26.91 MPa. From these results, even in a case where the thickness of the second resin layer is approximately 3 µm, the strength was improved significantly, which indicated that the second resin layer reinforced the first resin layer.

The above-described examples are only explanatory, and should not be understood as being limitative to the present disclosure. The present disclosure has been described using typical embodiments. The wordings used in the description and drawings of the present disclosure are to be understood not as being limitative but as being explanatory and exemplary. As described in detail here, modifications can be made within the scope of the appended claims without departing from the scope or nature of the present disclosure in the form. Although specific structures, materials and examples have been referred to herein in the details of the present disclosure, it is not intended to limit the present disclosure to the disclosures herein. The present disclosure shall rather cover all functionally equivalent structures, methods and uses within the scope of the appended claims.

### REFERENCE SIGNS LIST

- 10: catheter
- 11: hollow shaft (medical tube)
- 11A: distal end portion
- 11B: other part
- 12: distal tip
- 13: connector
- 15: lumen
- 20: first resin layer
- 21: inner peripheral surface
- 22: outer peripheral surface
- 30: second resin layer
- 32: outer peripheral surface
- 40: third resin layer
- 45, 145: reinforcing layer
- 45A, 45B, 145A: wire

## Claims

1. A medical tube, comprising:
a first resin layer that is configured to contain fluororesin; and
a second resin layer that coats an outer peripheral surface of the first resin layer and is configured to contain polyimide resin, wherein
a thickness of the second resin layer is larger than 0 µm but not exceeding 15 µm.

2. The medical tube according to claim 1, further comprising:
a third resin layer that coats an outer peripheral surface of the second resin layer, wherein
a thickness of the third resin layer is larger than the thickness of the second resin layer.

3. The medical tube according to claim 2, wherein the third resin layer includes an embedded reinforcing layer.

4. The medical tube according to any one of claims 1 to 3, wherein a distal end portion of the medical tube is more flexible than another part of the medical tube positioned on a more proximal end side than the distal end portion.

5. A catheter comprising the medical tube according to any one of claims 1 to 4.
